# EUROPEAN PATENT APPLICATION

(11) **EP 4 227 396 A1**
(43) Date of publication of application: **16.08.2023**
(21) Application number: 21880466.4
(22) Date of filing: 12.10.2021
(51) Int. Cl.: C12M 1/12, C12M 1/00, C12N 5/00, C12N 5/077, C12N 11/02

(54) **GELATIN MICROPARTICLES CONTAINING NUTRIENTS FOR CELL CULTURE THEREIN, PREPARATION METHOD THEREFOR, AND USE THEREOF**

(30) Priority: 12.10.2020 KR 20200131410
(71) Applicant: CJ Cheiljedang Corporation, Seoul 04560 (KR)
(72) Inventor: LEE, Hyeong Rae, Seoul 04560 (KR); LEE, Seung Yeon, Seoul 04560 (KR); BAEK, Seung Jin, Seoul 04560 (KR); HONG, Jin Kee, Seoul 03722 (KR); PARK, So Hyeon, Seoul 03722 (KR)
(74) Representative: Cabinet Laurent & Charras
(86) International application number: PCT/KR2021/014019
(87) International publication number: WO 2022/080819

(57) **Abstract**

The present application relates to gelatin microparticles containing nutrients for cell culture therein, a preparation method therefor, and a use thereof. The gelatin microparticles of the present application can be advantageously used in cell cultivation for preparing cultured meat.

## Description

### TECHNICAL FIELD

The present application relates to gelatin microparticles containing nutrients for cell culture therein, a preparation method therefor, and a use thereof.

### BACKGROUND ART

Cultured meat is artificial meat prepared through mass culture of cells without slaughter of livestock, and is prepared through an ethical and eco-friendly process, unlike actual meat that requires a process of breeding and slaughtering livestocks and a use of antibiotics. The process for preparing cultured meat is performed through the following process: First, muscle satellite cells are isolated from livestock, and then the isolated cells are mass-proliferated in a culture medium containing amino acids, vitamins, sugars, inorganic salts, minerals, and various growth factors in a bioreactor. The prepared cells are transferred to a specific support or differentiated into muscle tissues in the differentiated culture medium through self-assembly between cells.

Although many companies around the world are challenging the cultured meat industry, it is difficult to commercialize the cultured meat due to the complex production process and high production costs. Since the cost of the culture medium used in the mass proliferation process of muscle cells isolated from livestock accounts for at least 70% of the total production cost of cultured meat, research for reducing the cost of the culture medium is an indispensable factor. The conventional culture used for cell proliferation includes fetal bovine serum (FBS) containing various growth factors, cytokines, proteins, and the like essential for cell growth. However, since the use of animal-derived components is not suitable for the original purpose of cultured meat, there is a demand for developing a technology capable of excluding or reducing FBS.

### [PRIOR ART DOCUMENTS]

U.S. Patent Publication No. 2012/0225483

Korean Patent Laid-open Publication No. 10-2019-0054425

### DISCLOSURE OF THE INVENTION

### TECHNICAL PROBLEM

The present inventors have made efforts to develop a nutrient delivery platform capable of replacing or reducing animal-derived serum during cell culture for preparing cultured meat. As a result, the present inventors have proved experimentally that a cell proliferation effect superior to that of the case of using serum can be achieved through the continuous and stable release of nutrients by preparing cross-linked gelatin microparticles and incorporating the nutrients for cell culture in pores of the prepared gelatin microparticles, and applying the resultant microparticles in a cell culture medium, thereby completing the present application.

Therefore, an aspect of the present application provides gelatin microparticles for delivering nutrients for cell culture.

Another aspect of the present application provides a composition for cell culture including gelatin microparticles.

Further another aspect of the present application provides a method for preparing cultured meat, the method including culturing cells in a cell culture medium containing gelatin microparticles.

Yet another aspect of the present application provides a method for reducing serum components in a cell culture medium, the method including culturing cells in a cell culture medium containing gelatin microparticles.

Still another aspect of the present application provides a method for producing gelatin microparticles for delivering nutrients for cell culture.

Still yet another aspect of the present application provides a method for controlling the release of nutrients for cell culture from gelatin microparticles.

### TECHNICAL SOLUTION

In order to accomplish the object of the invention,

according to an aspect of the present application, there is provided gelatin microparticles including cross-linked gelatin microparticles and nutrients for cell culture incorporated in the particles.

According to another aspect of the present application, there is provided a composition for cell culture including the gelatin microparticles.

According to further another aspect of the present application, there is provided a method for preparing cultured meat, the method including culturing cells in a cell culture medium containing gelatin microparticles.

According to still another aspect of the present application, there is provided a method for reducing serum components in a cell culture medium, the method including culturing cells in the cell culture medium containing gelatin microparticles.

According to yet another aspect of the present application, there is provided a composition for preparing the gelatin microparticles.

According to still yet another aspect of the present application, there is provided a method for controlling the release of nutrients for cell culture from gelatin microparticles by controlling a degree of cross-linking of the gelatin microparticles.

Hereinafter, the present application will be described in detail.

According to an aspect, the present application provides gelatin microparticles which are formed by cross-linking gelatin and into which nutrients for cell culture are incorporated.

### Gelatin Microparticles

In the present application, the gelatin microparticles may be prepared by an emulsification method. For example, the gelatin microparticles may be prepared by a water-in-oil emulsification method for dispersing, in oil, a solution in which gelatin is dissolved.

The gelatin microparticles prepared by the emulsification method may go through a drying step. The dried gelatin microparticles may be cross-linked by a cross-linking reaction.

### Cross-linking of Gelatin Microparticles

The prepared gelatin microparticles may be cross-linked.

In an embodiment, the cross-linking of the gelatin microparticles may be performed in the presence of a cross-linking agent, and the cross-linking agent may be genipin.

The genipin may be a compound represented by Formula 1 below:

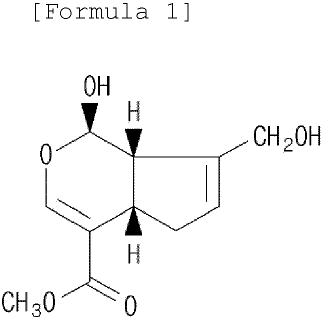

Cross-linking may be performed by adding the gelatin microparticles prepared by the emulsification method to a cross-linking agent solution prepared by dissolving a cross-linking agent in a solvent and then reacting the mixture.

The degree of cross-linking of the gelatin microparticles may be controlled by controlling the cross-linking reaction. For example, the degree of cross-linking may be controlled by controlling the concentration of the cross-linking agent, the concentration of the microparticles, or the reaction time.

In the present application, the degree of cross-linking of the gelatin microparticles may vary with the cross-linking reaction time, and may be 10-60%, 12-58%, 14-56%, 16-54%, 18-52%, 20-50%, 22-48%, 24-46%, 26-44%, 28-42%, or 30-40%.

In the present application, the degree of cross-linking of the gelatin microparticles may be expressed by the cross-linking reaction time.

In an embodiment, the degree of cross-linking of the gelatin microparticles may be obtained by reacting a cross-linking reaction solution containing a 90% ethanol solvent, a 0.5 w/v% concentration of genipin, and a 50 mg/mL concentration of dry gelatin microparticles for a reaction time of 2-60 hours, 3-58 hours, 4-56 hours, 5-54 hours, 5-52 hours, 5-50 hours, 5-49 hours, 5-48 hours, or 6-48 hours, more specifically, 6-16 hours, 4-8 hours, 5-7 hours, 10-14 hours, 11-13 hours, 14-18 hours, 15-17 hours, 22-26 hours, 23-25 hours, 40-46 hours, or 47-49 hours.

In the present invention, the loading amount and the release characteristics of nutrients for cell culture incorporated in microparticles vary with the degree of cross-linking of the gelatin microparticles so that the loading amount and the release characteristics of the nutrients may be controlled by controlling the degree of cross-linking of the microparticles.

In an embodiment, the cross-linking is carried out by the cross-linking agent, and a higher level of the cross-linking agent may be present on the surfaces of the gelatin microparticles compared to the insides of the gelatin microparticles. The cross-linking agent present on the surfaces of gelatin microparticles may have an effect on the loading amount and release rate of the nutrients.

In the present application, the gelatin microparticles prepared by the emulsification method and cross-linking may have a size of 1-200 µm, 3-170 µm, 5-150 µm, 5-120 um, 5-110 µm, 5-100 µm, 5-90 µm, 5-80 um, 5-70 µm, 6-70 µm, 7-70 µm, 8-70 um, or 10-50 µm.

### Incorporation of Nutrients for Cell Culture

In the present application, the gelatin microparticles cross-linked by the cross-linking agent may be added to the solution in which the nutrients for cell culture are dissolved, thereby incorporating the nutrients into the microparticles.

The nutrients for cell culture incorporated into the gelatin microparticles may be used without limitation as long as they are materials capable of forming a complex with gelatin through electrostatic attraction, ionic bonding, or hydrogen bonding.

The nutrients for cell culture that may be incorporated into the gelatin microparticles may be high molecular weight materials having a molecular weight of 1-50 kDa, 3-47 kDa, 5-45 kDa, 7-43 kDa, 10-40 kDa, 13-37 kDa, 15-35 kDa, 17-33 kDa, or 20-30 kDa.

In addition, the nutrients for cell culture that may be incorporated into the gelatin microparticles may be low molecular weight materials having 1 kDa or less. Specifically, the nutrients may be low molecular weight materials having a molecular weight of 0.01-1 kDa, 0.03-1 kDa, 0.05-1 kDa, 0.07-1 kDa, 0.07-0.95 kDa, 0.10-0.9 kDa, 0.15-0.90 kDa, 0.2-0.85 kDa, 0.25-0.8 kDa, or 0.3-0.75 kDa.

The nutrients for cell culture that may be incorporated into the gelatin microparticles may include hormones, growth factors, amino acids, or vitamins necessary for cell culture. For example, the nutrients for cell culture may be C-phycocyanin; albumin; growth factors selected from the group consisting of IGF-1, BMP-2, FGF-1, and VEGF; amino acids; or ascorbic acid, but are not limited thereto.

The gelatin microparticles of the present application as described above may be used for cell culture.

In an embodiment, the cells may be animal-derived muscle cells.

According to another aspect, the present application provides a composition for cell culture including: (i) gelatin microparticles which are formed by cross-linking gelatin and into which nutrients for cell culture are incorporated; and (ii) serum components.

In the composition for cell culture of the present application, the cross-linking may be carried out by genipin.

In the composition for cell culture of the present invention, the nutrients for cell culture may be C-phycocyanin, albumin; growth factors selected from the group consisting of IGF-1, BMP-2, FGF-1, and VEGF; amino acids; or ascorbic acid.

In the composition for cell culture of the present application, the degree of cross-linking of the gelatin microparticles may be 10-60%.

In the composition for cell culture of the present application, the degree of cross-linking of the gelatin microparticles may be obtained by reacting a cross-linking reaction solution containing a 90% ethanol solvent, a 0.5 w/v% concentration of genipin, and a 50 mg/mL concentration of dry gelatin microparticles for a reaction time of 2-60 hours, 3-58 hours, 4-56 hours, 5-54 hours, 5-52 hours, 5-50 hours, 5-49 hours, 5-48 hours, or 6-48 hours, more specifically, 6-16 hours, 4-8 hours, 5-7 hours, 10-14 hours, 11-13 hours, 14-18 hours, 15-17 hours, 22-26 hours, 23-25 hours, 46-40 hours, or 47-49 hours.

In the present application, the composition for cell culture may further include other components that may be included in the medium for cell culture, in addition to the gelatin microparticles containing the nutrients for cell culture as described above. The additional components may be, for example, carbon sources (e.g., glucose), minerals, amino acids, vitamins, lipids, serum, serum-derived components, or a combination of one or more thereof, but are not limited thereto.

The composition for cell culture of the present application may be contained in an amount in which the serum components are reduced.

In an embodiment, the serum component content may be less than 10 wt%, more specifically less than 9 wt%, less than 8 wt%, less than 7 wt%, less than 6 wt%, less than 5 wt%, or less than 4 wt% in the composition for cell culture.

The lower limit of the serum component content may be 0.001 wt%, 0.01 wt%, or 0.1 wt% in the medium for cell culture.

In an embodiment, the serum component may be fetal bovine serum, and the fetal bovine serum content may be less than 10 wt%, more specifically less than 9 wt%, less than 8 wt%, less than 7 wt%, less than 6 wt%, less than 5 wt%, and less than 4 wt% in the composition for cell culture, and the lower limit of the fetal bovine serum content may be 0.001 wt%, 0.01 wt%, or 0.1 wt% in the composition for cell culture.

Even through the composition of the present application contains the serum components having a content lower than 10 wt%, which is the content of serum components (e.g., fetal bovine serum) contained in a conventional cell culture medium, when the composition contains the above-described gelatin microparticles of the present application, equal or higher level of proliferation in cell proliferation, more specifically, muscle cell proliferation is possible.

The composition for cell culture of the present application may further include a serum-free medium, and for example, may include Dulbecco's Modified Eagle's Medium (DMEM), Eagle's Minimal Essential Medium (MEM), RPMI 1640, Ham's F 12, or SF 12 serum-free medium, or may further include some of the components of the medium.

In the composition for cell culture of the present application, the cells may be cells for preparing cultured meat.

In the composition for cell culture of the present application, the cells for preparing cultured meat may be animal-derived muscle cells, and the muscle cells may include myoblast, muscle satellite cells, myotube cells, myotubes, myofibers, or mature myofibers.

According to further another aspect, the present application provides a method for preparing cultured meat, the method including culturing cells in a cell culture medium containing gelatin microparticles which are formed by cross-linking and into which nutrients for cell culture are incorporated.

In the method for preparing cultured meat of the present application, the cross-linking may be carried out by genipin.

In the method for preparing cultured meat of the present invention, the nutrients for cell culture may be C-phycocyanin, albumin; growth factors selected from the group consisting of IGF-1, BMP-2, FGF-1, and VEGF; amino acids; or ascorbic acid.

In the method for preparing cultured meat of the present application, the degree of cross-linking of the gelatin microparticles may be 10-60%.

In the method for preparing cultured meat of the present application, the degree of cross-linking of the gelatin microparticles may be obtained by reacting a cross-linking reaction solution containing a 90% ethanol solvent, a 0.5 w/v% concentration of genipin, and a 50 mg/mL concentration of dry gelatin microparticles for a reaction time of 2-60 hours, 3-58 hours, 4-56 hours, 5-54 hours, 5-52 hours, 5-50 hours, 5-49 hours, 5-48 hours, or 6-48 hours, more specifically, 6-16 hours, 4-8 hours, 5-7 hours, 10-14 hours, 11-13 hours, 14-18 hours, 15-17 hours, 22-26 hours, 23-25 hours, 40-46 hours, or 47-49 hours.

In the method for preparing cultured meat of the present application, the cells may be animal-derived muscle cells, and the muscle cells may include myoblast, muscle satellite cells, myotube cells, myotubes, myofibers, or mature myofibers.

According to yet another aspect, the present application provides a method for reducing serum components in a cell culture medium, the method including culturing cells in a cell culture medium containing gelatin microparticles which are formed by cross-linking and into which nutrients for cell culture are incorporated.

In the method for reducing serum components of the present application, the cross-linking may be carried out by genipin.

In the method for reducing serum components of the present invention, the nutrients for cell culture may be C-phycocyanin, albumin; growth factors selected from the group consisting of IGF-1, BMP-2, FGF-1, and VEGF; amino acids; or ascorbic acid.

In the method for reducing serum components of the present application, the degree of cross-linking of the gelatin microparticles may be 10-60%.

In the method for reducing serum components of the present application, the degree of cross-linking of the gelatin microparticles may be obtained by reacting a cross-linking reaction solution containing a 90% ethanol solvent, a 0.5 w/v% concentration of genipin, and a 50 mg/mL concentration of dry gelatin microparticles for a reaction time of 2-60 hours, 3-58 hours, 4-56 hours, 5-54 hours, 5-52 hours, 5-50 hours, 5-49 hours, 5-48 hours, or 6-48 hours, more specifically, 6-16 hours, 4-8 hours, 5-7 hours, 10-14 hours, 11-13 hours, 14-18 hours, 15-17 hours, 22-26 hours, 23-25 hours, 40-46 hours, or 47-49 hours.

According to still another aspect, the present application provides a method for preparing gelatin microparticles for cell culture, the method including the following steps of: (a) preparing gelatin microparticles by an emulsification method; (b) reacting and cross-linking the prepared gelatin microparticles in the presence of a cross-linking agent; and (c) incorporating nutrients for cell culture into the cross-linked gelatin microparticles.

In the method for preparing gelatin microparticles for cell culture of the present application, the cross-linking agent may be genipin.

According to still yet another aspect, the present application provides a method for controlling the release of nutrients for cell culture, the method including the steps of: (a) preparing gelatin microparticles by an emulsification method; (b) reacting and cross-linking the prepared gelatin microparticles in the presence of a cross-linking agent; and (c) incorporating nutrients for cell culture into the cross-linked gelatin microparticles, wherein the release of nutrients for cell culture from the gelatin microparticles is controlled by controlling the degree of cross-linking in step (b) above.

In the other aspects of the present application, which are the composition for cell culture, the method for preparing culture meat, the method for reducing serum components in a cell culture medium, the method for preparing gelatin microparticles, and the method for controlling the release of nutrients for cell culture, the description of the gelatin microparticles is the same as described in the gelatin microparticles, which is an aspect of the present application, and thus is not described in duplicate.

### ADVANTAGEOUS EFFECTS

According to the gelatin microparticles of the present application, nutrients necessary for cell culture can be delivered stably.

According to the gelatin microparticles of the present application, the problems of ethics, high costs, and causing diseases caused by the use of serum can be solved due to the use of nutrients which can replace animal-derived serum.

The degree of cross-linking of the gelatin microparticles of the present application can be easily controlled, and thus the amount of nutrients incorporated, the amount of release, and the release speed can be controlled.

The present application is suitable for preparing artificial cultured meat due to the use of a gelatin and a cross-linking agent which are derived from a living organism, edible, and safe.

However, the effects of the present application are not limited to the above-mentioned effects and other effects not mentioned will be clearly understood from the following description by a person skilled in the art.

### BRIEF DESCRIPTION OF THE DRAWINGS

FIG. 1 is an optical microscope image showing the shape and size of gelatin microparticles (GMS) according to the degree of cross-linking.
FIG. 2A shows a mechanism of cross-linking gelatin particles by genipin, FIG. 2B shows graphs showing changes in Raman spectroscopy spectra due to the formation of a heterocyclic amine and the formation of an aromatic amide according to cross-linking by genipin, and FIG. 2C shows scanning electron microscope (SEM) images obtained by observing changes in shape of GMS according to the cross-linking time.
FIG. 3 shows the results of measuring the degradation rates of GMS samples cross-linked for 12 hours and 24 hours.
FIG. 4A is a graph (upper graph) obtained by analyzing a total accumulated release amount of C-PC in GMS samples cross-linked for 6 hours, 16 hours, 24 hours, and 48 hours, respectively, and a graph (lower graph) obtained by analyzing the release behavior of C-PC.
FIG. 4B is a graph obtained by analyzing the release behavior of IGF-1 in GMS cross-linked for 12 hours (12h_GMS).
FIG. 4C is a graph obtained by analyzing the release amount of IGF-1 incorporated into a GMS bare sample and GMS samples which are cross-linked for 6 hours, 16 hours, 24 hours, and 48 hours.
FIG. 5 is an experimental schematic diagram for evaluating the cell proliferation effect by applying gelatin microparticles, into which nutrients are incorporated, to cell culture.
FIG. 6 shows results of evaluation of proliferation of C2C12 cells by C-PC-incorporated GMS.
FIG. 7 shows results of evaluation of proliferation of C2C12 cells by IGF-1-incorporated GMS.
FIG. 8 shows graphs showing results of analyzing the release behavior of hyaluronic acid (HA) or FITC released from GMS into which HA or FITC is incorporated.

### MODE FOR CARRYING OUT THE INVENTION

Hereinafter, the present application will be described in detail with reference to examples. However, the following examples specifically illustrate the present application, and the contents of the present application are not limited by the following examples.

### Examples

### Example 1: Preparation of gelatin Microparticles (GMS) and Nutrient-incorporated Complex

Gelatin microparticles were prepared by a water/oil emulsion method. First, a 11.1 wt% gelatin (from Sigma-Aldrich) solution preheated at 45 °C was added dropwise in olive oil (from Sigma-aldrich) and the resultant mixture was stirred at 500 rpm for 10 minutes. After continuously stirring the mixture, the mixture was slowly cooled to 4 °C to be gelled, and after 30 minutes, 100 mL of cooled acetone was added thereto, mixed and stirred. After 1 hour, 100 mL of cooled acetone was further mixed and stirred at 1,000 rpm for 5 minutes. The generated microparticles were collected by filtration, washed with acetone to remove residual oil, and then naturally dried. A genipin (from Sigma-Aldrich) cross-linking solution having a concentration of 0.5 w/v% was prepared using 90% ethanol (Sigma-Aldrich) as a solvent, and dried microparticles (50 mg/mL) were added thereto and cross-linked. The degree of cross-linking was controlled by differently setting the cross-linking time to 6 hours, 12 hours, 16 hours, 24 hours, or 48 hours. The cross-linked gelatin microparticles (GMS) were washed three times with ethanol and then dried in an oven at 80 °C for 3 hours. Nutrients were incorporated by adding the dried GMS to a IGF-1 solution (0.5 µg/mL) or a C-phycocyanin (C-PC) solution (2 mg/mL) at a concentration of 10 mg/mL, and reacting the mixed solution for 12 hours.

### Example 2: Evaluation Experiment on Efficacy of Nutrient Delivery of Gelatin Microparticles

Gelatin microparticles into which nutrients were incorporated were applied to a cell culture environment to evaluate the efficacy of nutrient delivery. C2C12 myoblast (Passage 6) was inoculated into each well of a 24 well-plate by 3.5 × 10³ cells. The cell culture was performed using a culture medium in which 1% penicillin/streptomycin antibiotic (Gibco^{®} Life Technologies) and 10% or 5% FBS (WELGENE) are contained in Dulbecco's Modified Eagle Medium (DMEM, Gibco^{®} Life Technologies). In the cell proliferation enhancement experiment for GMS into which C-PC was incorporated, a culture medium containing 10% FBS was used in control group 1, a culture medium containing 5% FBS was used in control group 2, a culture medium containing 6-hour cross-linked GMS (GMS_6) into which C-PC was incorporated was used in experimental group 1, a culture medium containing 16-hour cross-linked GMS (GMS_16) into which C-PC was incorporated was used in experimental group 2, a culture medium containing 24-hour cross-linked GMS (GMS_24) into which C-PC was incorporated was used in experimental group 3, and a culture medium containing 48-hour cross-linked GMS (GMS_48) into which C-PC was incorporated was used in experimental group 4. The cell proliferation enhancement experiment for GMS into which IGF-1 was incorporated was also performed by setting a control group and an experimental group in the same manner as the experimental method for GMS into which C-PC was incorporated.

### Example 3: Method for Analyzing Characteristics of Gelatin Microparticles

The characteristic analysis of GMS was performed as follows. The roughness and fine shape of porosity of the GMS were observed using SEM. The color, shape, and swelling ratio of the GMS according to cross-linking were observed using an optical microscope (OM). The C-PC released from the GMS was detected using Photoluminescence spectroscopy (PL) and Micro reader to quantify the release amount over time and analyze the release behavior. The growth factor (IGF-1) released from the GMS was quantified using ELISA.

### Example 4: Analysis of Shape, Size and Degradation Rate of Gelatin Microparticles according to Degree of Cross-linking

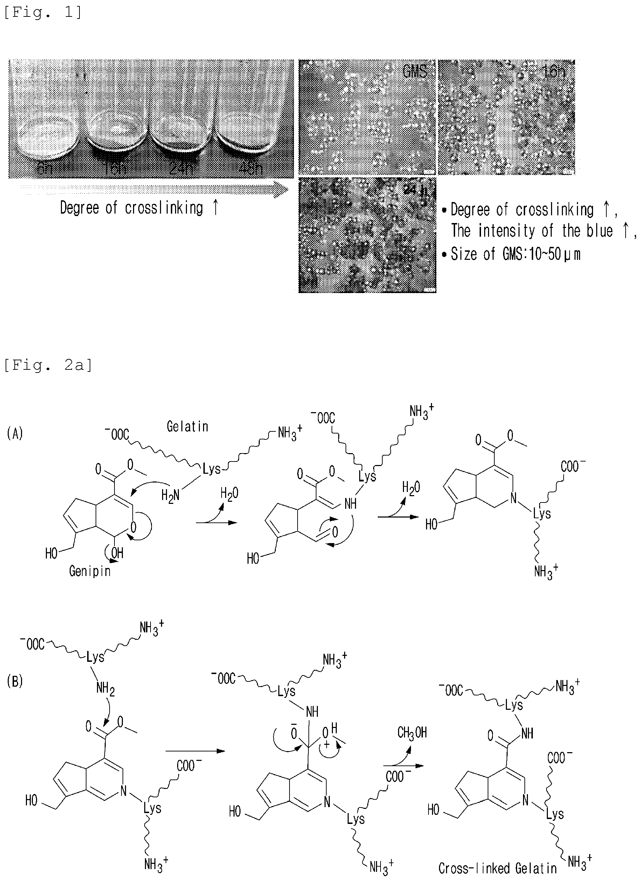

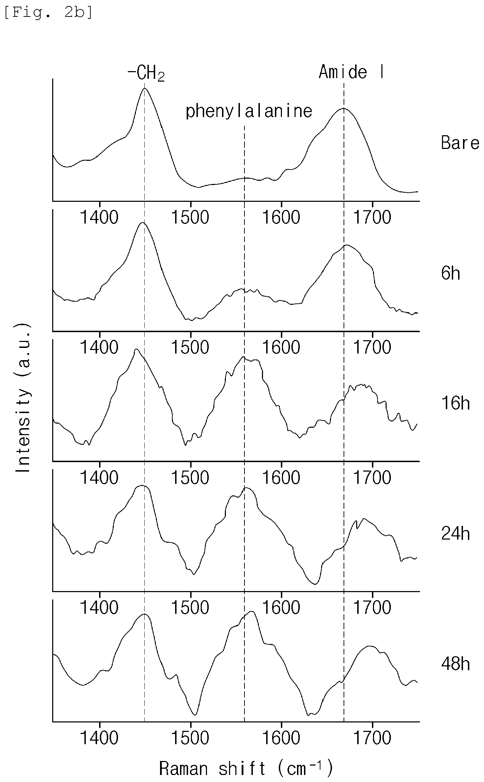

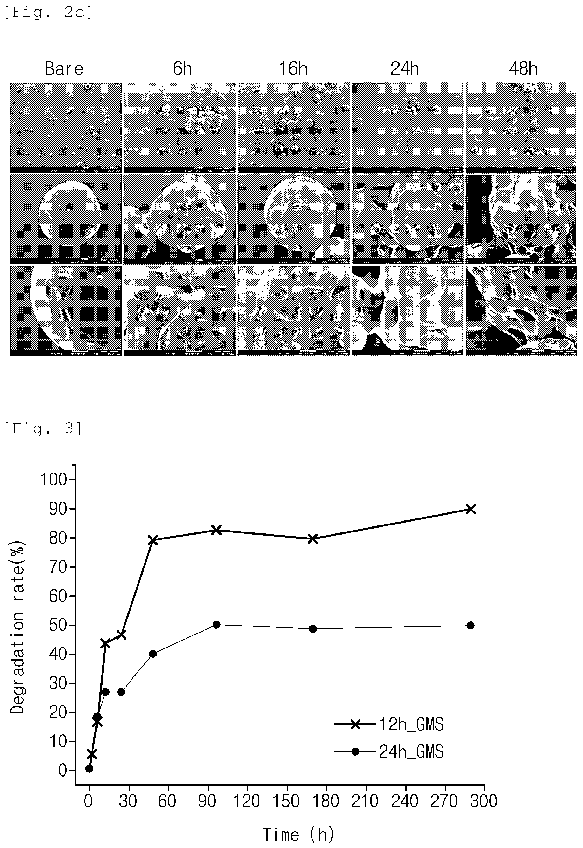

The size of the prepared GMS was observed to be 10-50 µm, and as the cross-linking time increased, the blue color of the GMS became darker and aggregation between the GMSs was observed (**FIG. 1**). It was confirmed that as the cross-linking was performed through Raman spectrum data, a new peak was formed around 1550 cm⁻¹ due to the formation of heterocyclic amine and aromatic amide, and shift of an amide I peak around 1680 cm⁻¹ occurred, thereby verifying that the cross-linking was successfully performed (**FIGS. 2A** **and** **2B**). In addition, a change in the shape of the GMS according to the cross-linking time was observed (**FIG. 2C**).

The rate of decrease in weight of the GMS in a cell culture environment for 12 days was measured to evaluate the degradation rate of each GMS. The weight of the GMS cross-linked for 12 hours (12h_GMS) was largely reduced within 48 hours and 90% or more of the GMS was degraded during the final 12 days. Considering that 7 days is required for the mass proliferation of cells for preparing cultured meat, and 5 days are required for the differentiation of cells, and thus a total period of 12 days for cell culture is required, the degree of degradation of 12h_GMS was suitable for preparing cultured meat. The weight of the GMS cross-linked for 24 hours (24h_GMS) was largely reduced within 96 hours and approximately 50% of the GMS was degraded during the final 12 days. For a total period of 12 days of cell proliferation and differentiation, the GMS was degraded by about half, indicating that nutrients will remain in the GMS after 12 days (**FIG. 3**).

As a result of the above experiment, it was proved that the degradation rate of the GMS may be controlled according to the degree of cross-linking, and thus, it was proved that the release amount and release rate of the incorporated nutrients may be controlled. Since the degradation rate of the GMS is closely related to the release of nutrients, the degradation rate decreases as the degree of cross-linking increases, and it is expected to release nutrients more slowly and continuously.

### Example 5: Analysis of Incorporation and Release of Nutrients in Gelatin Microparticles (GMS) according to Degree of Cross-linking

### 1) Analysis of Loading Efficiency of C-PC in GMS according to Degree of Cross-linking

The loading amount of C-PC in GMS according to the degree of cross-linking was analyzed. According to the experimental results shown in Table 1 below, the loading amount and efficiency of C-PC were in the order of 6h > Bare > 24h ≥ 16h > 48h. As the degree of cross-linking increases, the density of the GMS increases, and the swelling ratio decreases, and thus the C-PC-loading space decreases. In the loading efficiency measurement result, it was found that the Bare and 6h samples had a loading efficiency of 30% or more, and the others had 17-20%, and thus the GMS internal structure was rapidly cross-linked between 6h and 16h. Although the difference in time between 6h and 16h samples was only 10 hours, the difference in loading efficiency was 16%, but although the difference in time between 16h and 48h samples was 32 hours, the difference in loading efficiency was 3.3%, which was no significant difference. This is supposed to be the result of the accumulation of genipin molecules in the outer layer of the GMS and the bonding of C-PC to the outer part. The loading efficiency is higher at 6h GMS than at Bare. When the degree of cross-linking is low, the viscoelasticity of the GMS is increased so that C-PC is easily loaded, and the degradation of the GMS is minimized during the process of loading C-PC.

**[Table 1]**

| | Bare_GMS | 6h_GMS | 16h_GMS | 24h_GMS | 48h_GMS |
|---|---|---|---|---|---|
| Remaining volume (mL) | 0.34 | 0.34 | 0.42 | 0.41 | 0.42 |
| Remaining cone (mg/mL) | 1.577±0.083 | 1.462±0.00 2 | 1.608±0.035 | 1.624±0.00 9 | 1.663±0.02 2 |
| Remaining amount (µg) | 736.04±28.2 8 | 697.18±0.8 7 | 875.25±14.8 4 | 865.87±3.8 0 | 811.62±9.5 9 |
| Loading amount (µg) | 363.96 | 402.82 | 224.75 | 234.13 | 188.38 |
| Loading efficienc y (%) | 33.09±2.57 | 36.62±0.08 | 20.43±1.35 | 21.28±0.35 | 17.13±0.87 |

### 2) Analysis of Release Behavior of C-PC in GMS

The GMS samples, which were cross-linked for 6, 16, 24, and 48 hours, were prepared in 4 mg each, C-PC was incorporated as a nutrient, and the release behavior for 7 days was then analyzed. By cross-linking, the interior of the GMS has a denser microstructure, which was expected to have an effect on the incorporation and release amounts of C-PC of 30 kDa. A first step of the release of C-PC from the GMS is a step of rapidly releasing a large amount of materials due to initial diffusion according to a concentration gradient. A second step of the release is a step of gradually and continuously releasing the incorporated materials by the degradation of the GMS.

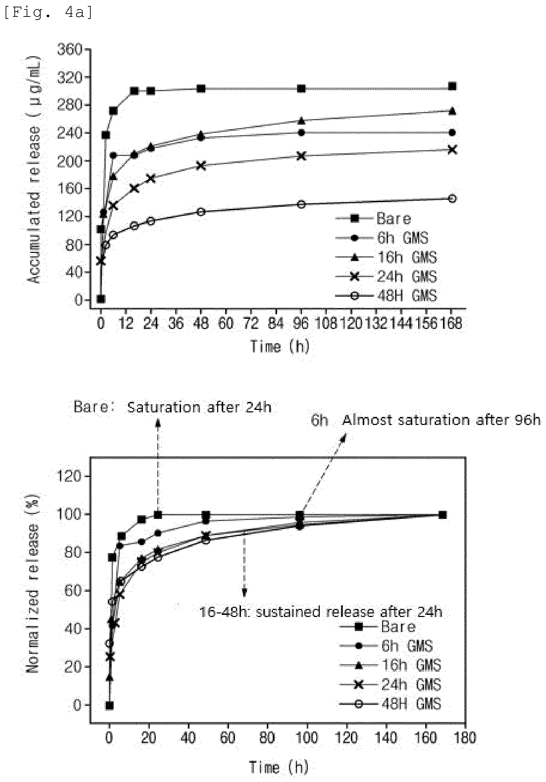

As a result of the experiment, the release amount (µg) was in the order of Bare > 16h ≥ 6h > 24h > 48h. The Bare GMS was saturated by both the diffusion of C-PC and the degradation of the GMS based on 24h. The 6h_GMS had a release rate of C-PC slower than the Bare due to the internal diffusion space reduced by some cross-linking, but after 48h, 95% of the 6h_GMS was released and the 6h_GMS was almost saturated at 96h. In the case of 16h to 48h GMSs, the loading amount between samples was not significantly different, and the release behavior was almost similar, but the release amount showed a distinct difference, and was higher in the order of 16h > 24h > 48h. From the case where the cross-linking time is greater than 24 hours, the inner structure of the GMS is cross-linked as well as genipin starts to be accumulated in the outer layer, and the interaction between the GMS surface and C-PC, or the interaction between C-PC and C-PC is enhanced, and thus the release rate is delayed (**FIG. 4A**).

### 3) Analysis of Release Behavior of IGF-1 in GMS

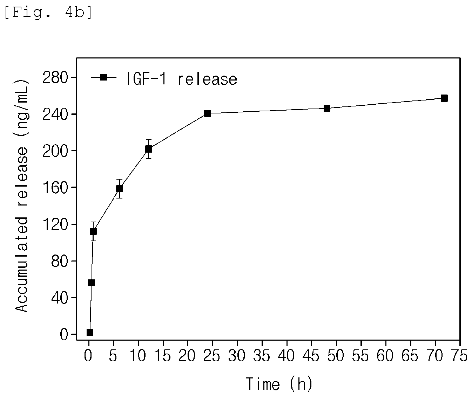

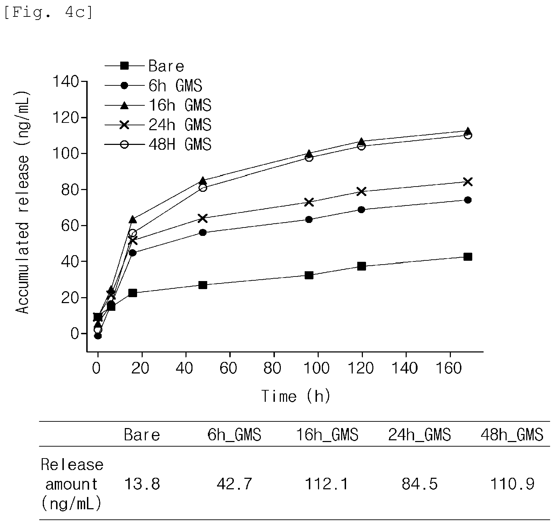

The release amount and release rate of IGF-1 were evaluated through ELISA quantitative analysis. During the initial 24 hours, rapid release occurred due to the diffusion phenomenon by the concentration gradient in and out of the GMS, and after 24 hours, IGF-1 was slowly released by slow degradation of the GMS. After 72 hours, the release of IGF-1 was sustained, and the maximum release amount was 256 ng/mL, which met the effective concentration for enhancing the proliferation of C2C12 cells (**FIG. 4B**). The release amount (ng/mL) of IGF-1 according to the degree of cross-linking was in the order of Bare < 6h < 24h < 48h ≒ 168h (FIG. 4C).

### Example 7: Evaluation of Efficacy of Gelatin Microparticles Incorporated with Nutrients on Cell Proliferation

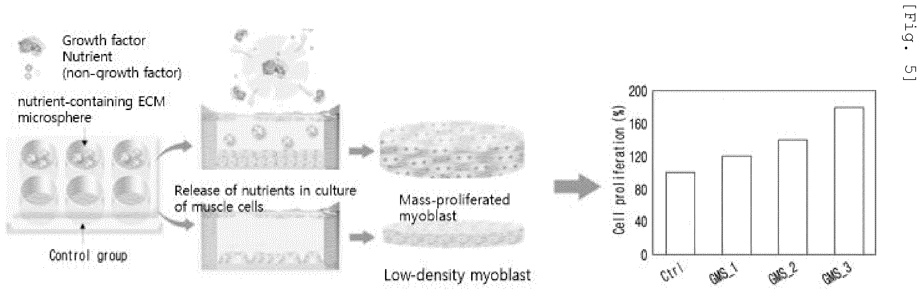

The cell proliferation effect was evaluated by applying the nutrient-incorporated gelatin microparticles to cell culture (**FIG. 5**).

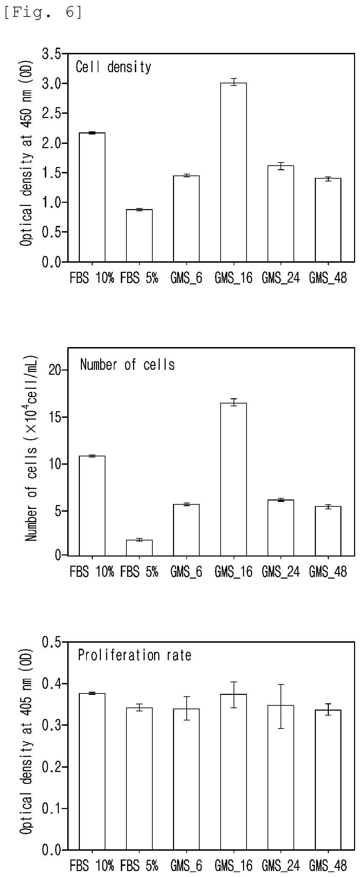

In general, Fetal Bovine Serum (FBS) is added to the cell culture medium at a concentration of 10%. In this Example, in order to evaluate the effect of FBS reduction due to the delivery of nutrients, the cell proliferation evaluation was performed by preparing a conventional medium (FBS 10%), a medium (FBS 5%) in which FBS was reduced, and media in which GMSs into which C-PC was incorporated and which were prepared by varying the degree of cross-linking in the FBS 5% medium were added, respectively. As a result of the experiments, when C-PC was released from the GMS cross-linked for 16 hours (GMS_16), the effect was the most obvious, and the number of cells increased by 1.6 times compared to the conventional medium (FBS 10%) despite the use of medium with reduced FBS amount. In addition, the number and density of cells in all groups to which GMS was added in addition to GMS_16 were higher than those of FBS-reduction medium (FBS 5%) (**FIG. 6**).

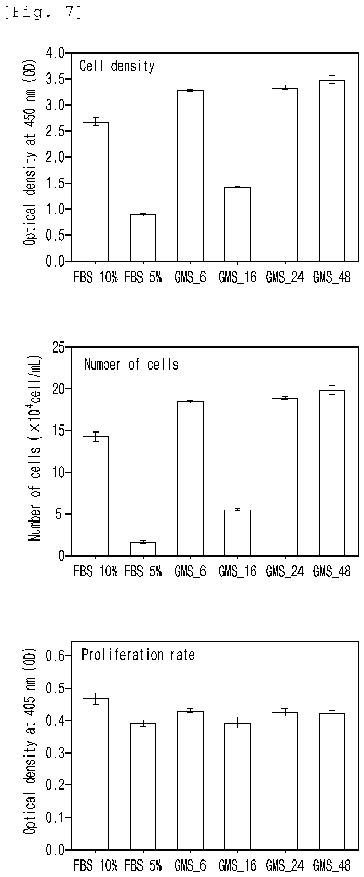

In the same manner as in the method of evaluation of proliferation of C-PC-incorporated GMS samples for C2C12 cells, the cell proliferation evaluation was performed by preparing a conventional medium (FBS 10%), a medium (FBS 5%) in which FBS was reduced, and media in which GMSs into which IGF-1 was incorporated and which were prepared by varying the degree of cross-linking in the FBS 5% medium were added, respectively. As a result of the experiments, the cell density, number of cells, and proliferation rate in the GMS-added group were significantly improved compared to the FBS 5%, and in particular, the 6h_GMS, 24h_GMS, and 48h_GMS groups were measured to have 1.4 times more cells than the FBS 10% group (**FIG. 7**).

It was confirmed through the experimental results that the amount of FBS used may be reduced when the nutrient-incorporated GMS is used.

### Example 8: Analysis of Release Behavior of High or Low Molecular Material-incorporated Gelatin Microparticles

The GMS can incorporate various materials by swelling properties and charged amino acid chains. In the actual process for preparing cultured meat, low-molecular materials in addition to C-PC and IGF-1 or various types of growth factors may be used. Hyaluronic acid (HA) as a high molecular material and Fluorescein isothiocyanate (FITC) as a low molecular material were selected as model materials in addition to IGF-1 and C-PC as materials incorporated into the GMS. The release behavior of the model materials was analyzed for the GMS samples into which HA or FITC was incorporated for 72 hours in media for culturing C2C12 cells.

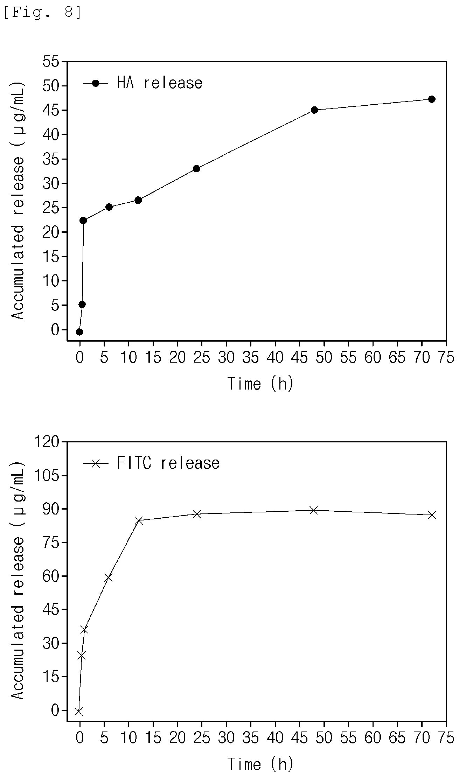

The HA was mixed with HA conjugated with rhodamine B dye at a ratio of 1:9 and used, and the release behavior was analyzed through photoluminescence (PL) measurement. The HA has a relatively high negative charge density in a neutral solution condition, and thus strong electrostatic attraction may act between the HA and the GMS positively charged. Accordingly, the GMS/HA interaction > diffusion rate was shown, and the part affected by diffusion was reduced, and the HA was slowly released as the GMS was degraded. Unlike IGF-1, C-PC, and HA, FITC is a low-molecular material model and the release behavior was analyzed by PL fluorescence analysis. In the case of the FITC, since the molecular weight is much smaller than the GMS, rapid release occurs depending on diffusion force rather than interaction with the GMS (**FIG. 8**).

Through the above results, it was proved that the nutrients having various functional groups or molecular weights required for the preparation of cultured meat may be incorporated into edible GMS and efficiently released.

Although the representative embodiments of the present application have been exemplarily described, the scope of the present application is not limited to the specific embodiments as described above, and a person skilled in the art can change the present application within the scope described in the claims of the present application.

## Claims

1. Gelatin microparticles which are formed by cross-linking gelatin and into which nutrients for cell culture are incorporated.

2. The gelatin microparticles of claim 1, wherein a cross-linking agent is present at a higher level on the surfaces of the gelatin microparticles than on the insides of the gelatin microparticles.

3. The gelatin microparticles of claim 1, wherein the cross-linking is carried out by genipin.

4. The gelatin microparticles of claim 1, wherein the nutrients for cell culture are C-phycocyanin, albumin; growth factors selected from the group consisting of IGF-1, BMP-2, FGF-1, and VEGF; amino acids; or ascorbic acid.

5. The gelatin microparticles of claim 1, wherein the degree of cross-linking of the gelatin microparticles is 10-60%.

6. The gelatin microparticles of claim 1, wherein the degree of cross-linking of the gelatin microparticles is obtained by reacting a cross-linking reaction solution containing a 90% ethanol solvent, a 0.5 w/v% concentration of genipin, and a 50 mg/mL concentration of dry gelatin microparticles for a reaction time of 2-60 hours.

7. The gelatin microparticles of claim 1, wherein the gelatin microparticles are used for cell culture.

8. The gelatin microparticles of claim 7, wherein the cells are animal-derived muscle cells.

9. A composition for cell culture comprising:
(i) the gelatin microparticles of any one of claims 1 to 6; and
(ii) serum components.

10. The composition of claim 9, wherein the serum components are contained in an amount of less than 10 wt% in the composition for cell culture.

11. A method for preparing cultured meat comprising culturing cells in a cell culture medium containing gelatin microparticles of any one of claims 1 to 6.

12. The method of claim 11, wherein the cells are animal-derived muscle cells.

13. A method for reducing serum components in a cell culture medium, the method comprising culturing cells in a cell culture medium containing gelatin microparticles of any one of claims 1 to 6.

14. A method for preparing gelatin microparticles for cell culture comprising the steps of:
(a) preparing gelatin microparticles by an emulsification method;
(b) reacting and cross-linking the prepared gelatin microparticles in the presence of a cross-linking agent; and
(c) incorporating nutrients for cell culture into the cross-linked gelatin microparticles.

15. The method of claim 14, wherein the cross-linking agent is genipin.

16. A method for controlling the release of nutrients for cell culture, the method comprising the steps of:
(a) preparing gelatin microparticles by an emulsification method;
(b) reacting and cross-linking the prepared gelatin microparticles in the presence of a cross-linking agent; and
(c) incorporating nutrients for cell culture into the cross-linked gelatin microparticles,
wherein the release of nutrients for cell culture from the gelatin microparticles is controlled by controlling the degree of cross-linking in step (b) above.
